# EUROPEAN PATENT APPLICATION

(11) **EP 3 342 353 A1**
(43) Date of publication of application: **04.07.2018**
(21) Application number: 16207439.7
(22) Date of filing: 30.12.2016
(51) Int. Cl.: A61B 10/00, A61F 5/56

(54) **MOUTHPIECE FOR COLLECTING ORAL FLUID**

(71) Applicant: Nokia Technologies Oy, 02610 Espoo (FI)
(72) Inventor: BAILEY, Marc, Cambridge CB4 3DD (GB); LONGBOTTOM, Simon, San Francisco, CA 94123 (US)
(74) Representative: Nokia EPO representatives

(57) **Abstract**

A mouthpiece, comprising at least one inlet configured to allow oral fluid from a user's oral cavity to travel into the mouthpiece; and a reservoir comprising one or more chambers, the reservoir configured to store oral fluid received from the user's oral cavity through the at least one inlet, wherein the mouthpiece is configured to be contained substantially within the user's oral cavity during the collection of oral fluid.

## Description

### TECHNICAL FIELD

The present application relates generally to a mouthpiece for collecting oral fluid.

### BACKGROUND

Oral fluid is a term used to describe a mixture of different substances in the oral cavity of a living being such as a person or animal. Typically oral fluid will include saliva, oral mucosal transudate, mucus from the respiratory tract, and contaminants such as traces of food and drink.

Oral fluid testing is an important diagnostic technique and be used to test for a variety of infections, hormonal imbalances, drug consumption, and other altered health states.

Oral fluid testing has many advantages such as ease of collection, safety, and non-invasiveness, when compared to other forms of sample collection such as venepuncture.

### SUMMARY

A first example provides a mouthpiece, comprising: at least one inlet configured to allow oral fluid from oral fluid from a user's oral cavity to travel into the mouthpiece; and a reservoir comprising one or more chambers, the reservoir configured to store oral fluid received from the user's oral cavity through the at least one inlet, wherein the mouthpiece is configured to be contained substantially within the user's oral cavity during the collection of oral fluid.

A second example provides a method of manufacturing a mouthpiece, the method comprising: providing at least one inlet configured to collect oral fluid from a user's oral cavity; providing a reservoir comprising one or more chambers, the reservoir being internal to the mouthpiece and configured to store oral fluid collected by the at least one inlet; and configuring the mouthpiece to be contained substantially within the user's oral cavity during the collection of oral fluid.

### BRIEF DESCRIPTION OF THE DRAWINGS

For a more complete understanding of example embodiments of the present invention, reference is now made to the following descriptions taken in connection with the accompanying drawings in which:
Fig. 1 illustrates a cross section of an example of a mouthpiece.
Fig. 2 illustrates a cross section of one side of another example of a mouthpiece.
Fig. 3 illustrates a cross section of one side of another example of a mouthpiece.
Fig. 4a illustrates another example of a mouthpiece.
Fig. 4b illustrates a cross section of the mouthpiece of Fig. 4a.
Fig. 4c illustrates another example of a mouthpiece.
Fig. 5a represents an example of a reservoir for a mouthpiece.
Fig. 5b and Fig. 5c illustrate a compression valve of the reservoir of Fig. 5a shown respectively in its closed and open configurations.
Figs. 6a-6c illustrate examples of chamber configuration within a reservoir for a mouthpiece.
Fig. 7 illustrates another example of a mouthpiece.
Fig. 8 illustrates a method of manufacturing a mouthpiece.

### DETAILED DESCRIPTON OF THE DRAWINGS

Oral fluid collected from the mouth or oral cavity of a living being may be processed in place and/or processed in a laboratory to, for example, analyse to identify markers of endocrine, immunologic, inflammatory, infectious, and other types of conditions. Saliva can be analysed to determine levels of biomarkers such as steroid hormones. For example, the levels of cortisol in saliva are known to closely correlate with levels of physiological stress.

The analysis of saliva and other oral fluid is made complicated by the presence of contaminants, for example traces of food and drink that have remained in the mouth. These contaminants can invalidate or compromise measurements taken on saliva samples. Such contamination may be minimised by collecting saliva first thing in the morning, for example, before eating, or by collecting saliva during the day before mealtimes. For some living beings, the relative levels of biomarkers in oral fluid change during the course of a user's day, and even without the presence of food and drink contaminants the level of cortisol (for example) may be significantly different if measured overnight whilst a user is asleep, compared to during the day when the user is awake and active. As used herein, the term "user" refers to a living being that has the capability to use a mouthpiece or be fitted with a mouthpiece.

Sometestscan be performed using a single drop of saliva, but clinical accuracy for some tests may require the collection of a larger volume of saliva. Saliva maybe collected by, for example, placing a cotton wool under the tongue, let it saturate with saliva and then remove. The saliva can be extracted from the cotton wool in the laboratory, for example, and tested using methods appropriate to the particular test. However, the extraction of saliva from cotton wool is not completely effective and may introduce errors into subsequent tests. For example, components for analysis may bind to the cotton wool so that the levels in the extracted saliva are artificially low (research indicates that this is true of cortisol, for example). Different media for absorbing oral fluid samples may be used with a reduced likelihood of adulteration of the sample, but are typically less comfortable for the user or have undesirable safety issues when placed in direct contact with the soft tissues inside the mouth. Cotton wool is a compromise as it can be placed safely and comfortably within the mouth.

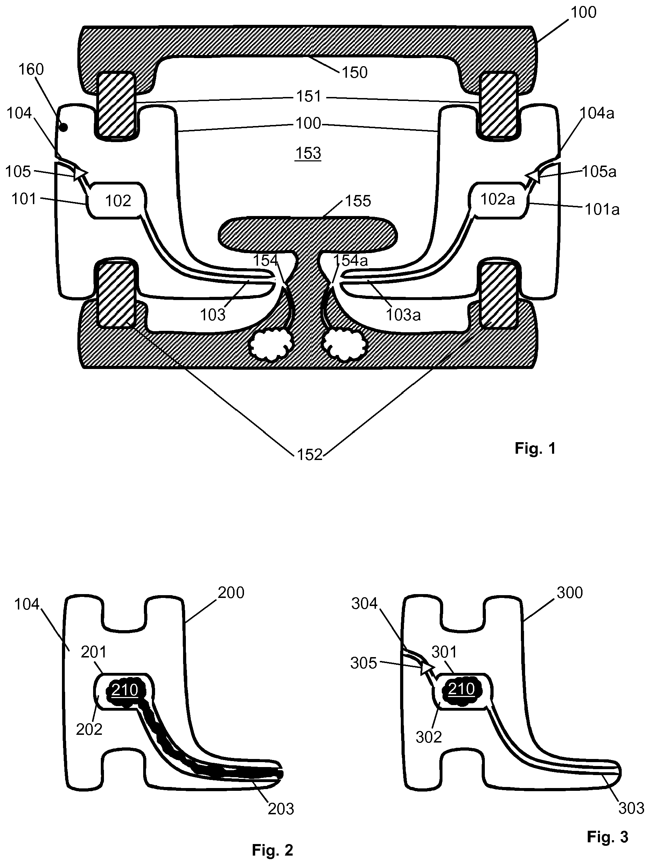

Fig. 1 shows a cross section of a mouthpiece 100 that can be worn by a user to collect a sample of oral fluid. It may be used to collect a sample that is predominantly saliva. The mouthpiece 100 may be made of a soft plastics material. Mouthpiece 100 may be moulded to conform to the shape of a user's upper 151 and/or lower 152 teeth. The mouthpiece 100 may be shaped to form an interference fit with a user's upper and/or lower teeth in order that it is held securely in position whilst in use. Alternatively, the mouthpiece 100 may be made of any other suitable material or any combination of materials, and may be held in place in any other suitable way. For example, the mouthpiece may be made of a hard plastics material, and/or may be held in place using an adhesive attaching an upper surface of the mouthpiece (not shown) to the bridge 150 of the user's oral cavity.

The mouthpiece 100 may serve to protect the user's teeth from damage caused by grinding and/or clenching, for example due to bruxism, since in use it will lie between the teeth 151 of the upper jaw and those 152 of the lower jaw. Bruxism is a parafunctional activity relating to excessive teeth grinding or jaw clenching, for example during sleep. Sufferers of bruxism may wear a mouthguard or mouthpiece when sleeping in order to minimise tooth damage. Such mouthguards may be solidly moulded from soft plastics material and provide a protective barrier between the teeth of the upper and lower jaws.

In use the mouthpiece 100 is enclosed entirely or at least substantially within the oral cavity of the user during the collection of oral fluid. For example, a majority of the mouthpiece 100 may be positioned within the oral cavity of the user with a small potion extending between the user's lips in order to assist in removing the mouthpiece 100 hygienically, or to enable the mouthpiece to be secured to the user's clothing to prevent it being lost should the user unintentionally remove it whilst he sleeps. However, despite a small extension of such a mouthpiece 100 outside the user's oral cavity when in use, the functional parts of the mouthpiece 100 used to collect and store a sample of oral fluid would all be contained entirely within it. This is in contrast to saliva extraction systems that have a mouthpart that is inserted into the oral cavity of a user with a storage system external to the user's oral cavity that is connected to the internal mouthpart by a conduit for saliva to flow to the external storage system - the majority of such a system remains outside the user's body during oral fluid collection and cannot be said to be contained substantially within the user's oral cavity.

The mouthpiece 100 comprises at least one reservoir 101 or 101 a that in turn comprises at least one chamber 102 or 102a. The mouthpiece 100 illustrated comprises two reservoirs 101 and 101 a, one on each side of the user's oral cavity. Each of these reservoirs 101 and 101 a is shown as comprising a single chamber 102 or 102a and each has its own inlet 103or 103a. In some examples, mouthpiece 100 may also include outlet 104 and/or 104a. Each outlet 104or 104a may have a one way valve 105or 105a. The two reservoirs may or may not be interconnected. In some examples, the two reservoirs are not interconnected, and the inlets and outlets are therefore specific to a single reservoir.

The mouthpiece of Fig. 1 is just one of many possible examples. In other examples the mouthpiece may comprise different numbers of reservoirs and/or chambers and these may be arranged in different configurations. The mouthpiece of Fig. 1 is symmetrical and each side is constructed and operates similarly. However, in other examples two sides may not be similar. For example, the two sides may contain different numbers and types of reservoir, for example one single-chamber reservoir on one side and no reservoir at all on of the other side.

The operation of just the left side of Fig. 1 will be described, for simplicity. As mentioned above, the two sides of this example are similar and operate in the same manner.

The chamber 102 of the reservoir 101 is configured to store oral fluid (e.g. saliva) that is collected by inlet 103 of the mouthpiece. In some examples, the inlet 103 extends between the outer surface of the mouthpiece 100 and the chamber 102 so as to provide for fluid travel between the user's oral cavity 153 and the chamber 102. For example, oral fluid may travel to the chamber 102 by capillary action and/or by the movement of the user, such as movement of the user's mouth or inside the oral cavity. The inlet 103 is positioned such that when the mouthpiece 100 use it extends to a position within the user's oral cavity that has been determined to be suitable for oral fluid collection. In the example of Fig.1 the inlet 103 is positioned to be proximate or near one of the sublingual salivary ducts 154154a, located underneath the tongue 155. The sublingual salivary ducts 154 and 154a convey saliva from the sublingual salivary glands 156 and 156a to the oral cavity 153.

In other examples (not shown), one or both of the inlet 103 and 103a may be positioned to collect saliva from different ducts and/or different locations in the oral cavity 153. In an example where an inlet 103 or 103a is not extends near salivary duct 154 or 154a, the portion of the mouthpiece 100 extended toward salivary duct 154 or 154a is not needed. For example, the inlet 103 may instead connect the chamber 102 to a different location of the mouthpiece 100 where it will be proximate one or more other glands, such as a submandibular salivary duct, in order to collect saliva from one or more of the other glands (e.g., submandibular salivary glands near a location 160 near the exterior of the mouthpiece 100).

In Fig. 1, two chambers 102, two inlets 103, two outlets 104, and two valves 105 are shown only as examples. In other examples (not shown), a mouthpiece 100 may include any number of chambers 102, any number of inlets 103, and any number of outlets 104. A mouthpiece 100 may include any number of one way valve (not shown) associated with inlets 103, independently of the number of inlets 103, and any number of one way valves 105 associated with outlets 104, independently of the number of outlets 104.

The chamber 102 may be deformable and a biting action on the mouthpiece 100 may cause it to be compressed. The compression of the chamber 102 may cause at least a portion of its contents to be purged from the chamber through its outlet 104, from where the contents is emptied into the user's oral cavity 153 (e.g., away from salivary gland 156). The outlet 104 may be provided with a one way valve that allows material such as oral fluid or air to pass through the outlet 104 in a direction towards the exterior of the mouthpiece 100 but prevents such material from entering the chamber through the outlet 104.

When the user bites down on the chamber 102 (and in doing so purges the reservoir 102) then subsequently releases his bite, the chamber 102 is permitted to return to its original un-compressed shape causing the pressure to reduce within the chamber 102 and oral fluid to be drawn into the chamber 102 through the inlet 103. Thus, natural biting/chewing motions of the user (e.g. during an episode of bruxism) can be used to pump oral fluid such as saliva into the chamber 102. Continued biting/chewing of the mouthpiece 100 (e.g. over the course of a night) will serve to continuously replace the oral fluid contained within the chamber 102, ensuring that the sample is fresh when the mouthpiece 100 is eventually removed (e.g. in the morning).

In some examples the inlet 103 may be also provided with a one way valve (not shown) that permits oral fluid to flow from the oral cavity 153 towards the chamber 102 but not from the chamber 102 towards the oral cavity 153. For example, the one way valve in the inlet 103 may normally be in an open position unobstructing movement of fluid or air until chamber 102 is compressed, which causes fluid or air in inlet 103 to travel in the reverse direction away from chamber 102. The movement fluid or air starts traveling in the reverse direction causes the one way valve in inlet 103 to be closed. Doing so will prevent old oral fluid samples contained within the chamber from being purged from the reservoir into the inlet 103 and into the vicinity of the inlet 103 in the oral cavity 153. Therefore, when the user ceases to bite down on the mouthpiece 100 the oral fluid that is drawn into the inlet 103 will be fresh oral fluid and not the previously purged contents of the reservoir 102. In other examples where the inlet 103 does not comprise a one way valve, the outlet 104 may have a diameter that is greater than the inlet 103 so that compression of the reservoir 102 will cause its contents to be purged predominantly via the outlet 104 with only a small amount leaving the reservoir 102 via the inlet 103.

The outlet 104 may be positioned towards the top of the chamber 102 according to an expected orientation of the mouthpiece 100 in use (e.g. when the mouthpiece 100 is correctly positioned within the user's oral cavity and the user is lying down). When the mouthpiece 100 is first placed in the user's oral cavity the chamber may contain air and placing the outlet towards the top of the chamber causes the air to be effectively purged through the outlet 104 during compression of the chamber.

Mouthpiece 100 can be designed to be worn in a time period as sort as a few seconds, one minute, a few minutes, etc. Once a suitable sample of oral fluid has been collected in the reservoirs 102and 102a, the user may remove the mouthpiece 100 from his oral cavity. Alternatively, the mouthpiece 100 can be designed to be left in the user's oral cavity until such time as it is convenient to remove - for example when the mouthpiece 100 is used overnight it may be removed by the user on waking.

The samples collected by the mouthpiece 100 may be used for chromatographic testing. Chromatography is the separation of a mixture by passing it in solution or suspension or as a vapour (in gas chromatography) through a medium in which the components move at different rates. Some chromatography media permit molecules of interest to bind to their surface in order to concentrate it and so increase the accuracy and ease of measurement. Different surfaces have different chemical properties which change which molecules bind and different analytical tests depend on this selective binding to the surface.

A reservoir in the mouthpiece 100 may include one or more chambers that each contain one or more binding media for selectively binding molecules that are of interest for use in chromatographic testing that depends on this selective binding.

Analysis of the oral fluid collected by the mouthpiece 100 may then take place. The user may, for example, deliver the mouthpiece 100 to a laboratory or other suitable remote location for testing of the collected oral fluid. It is not necessary for the user to drain the oral fluid out of the mouthpiece 100 (e.g. by squeezing it to deform its chambers 102and 102a and purge the reservoir), although he may do so and decant the oral fluid to another vessel. Instead, he may provide the entire mouthpiece for analysis. The user may be provided with a case for the mouthpiece 100 that is designed to the analysis of the sample, for example by cooling the mouthpiece 100 in order to maintain the integrity of the oral fluid sample, or containing a preservative solution that can be pumped into the mouthpiece. Alternatively the case may include a sample extraction system for removing the sample from the mouthpiece 100, for example by pumping. The case may include suitable sensors for analysing the sample, for example by detecting the presence of biomarkers in the oral fluid when it is removed from the reservoirs of the mouthpiece. Although described here in the context of a case for the mouthpiece, such a system may not be provided in the form factor of a case, and may take any suitable form.

The example shown in Fig. 1 includes two reservoirs 101 and 101 a, each of which comprises a single deformable chamber 102 or 102a which may be compressed and released in order to draw in oral fluid through the inlets 103and 103a. The mechanism for collecting oral fluid as described with Fig. 1 is only an example. Any mechanisms may be used, and other mechanisms are also possible.

Fig. 2 illustrates a cross section of a portion of a different example of a mouthpiece 200 in which an outlet may not be present (no outlet is shown). The chamber 202 of the reservoir 201 mouthpiece 200 is not necessarily compressible and may instead be rigid or semi-rigid. A rigid or semi-rigid chamber has the advantage that media located inside the chamber to assist in retaining oral fluid or to influence the collected sample in some way (e.g. by selectively binding certain molecules in the sample) can be protected from compression by the user's teeth. The chamber 202 and inlet 203 of Fig. 2 contain an absorbent wicking material 210 such as cotton wool, gauze or artificial sponge that wicks oral fluid through the inlet 203 and into the chamber where it is stored. Properties including dimensions, density of packing, and type of wicking material 210 and dimensions of the inlet 203 and chamber 202 can be chosen such that a desired volume of oral fluid is wicked into the chamber over the course of a desired sample time by capillary flow through the wicking material 210. For example, they may be chosen such that the wicking material 210 becomes saturated with oral fluid only after a number of hours of continued use of the mouthpiece 200.

Fig. 3 illustrates a cross section of a portion of different example of a mouthpiece 300 that includes both an inlet 303 and an outlet 304. In the illustrated example the outlet 204 includes a one-way valve permitting flow only from the reservoir 301 towards the oral cavity, but this valve need not necessarily be present. As for the example of Fig. 2, the chamber 302 of the reservoir 301 may be rigid or semi-rigid. In this example no wicking material is provided, but instead the dimensions of the inlet 303 are chosen such that the inlet is sufficiently narrow to cause oral fluid to enter the chamber 302 through capillary flow through the inlet. An absorbent material 302 such as cotton wool may be located within the chamber 302 in order to retain oral fluid drawn into the chamber 302 through the inlet 303.

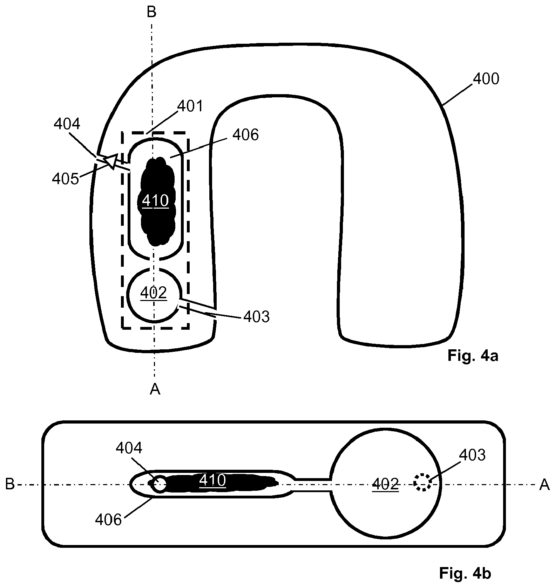

In some examples a single reservoir may comprise a plurality of chambers. Fig. 4a illustrates such an example where a mouthpiece 400 comprises an inlet 403, an outlet 404 with a valve 405 and a reservoir 401 comprising a first chamber 402 and a second chamber 406. The inlet 403 is connected to the first chamber 402 and the outlet 404 is connected to the second chamber 406. The first and second chambers are connected to one another so that oral fluid can pass from the user's oral cavity 153 into the inlet 403, from the inlet 404 into the first chamber 402, from the first chamber into the second chamber 406, from the second chamber 406 into the outlet 404 and through the one-way valve 405, and finally return from the outlet 404 back into the oral cavity 153. The first chamber 402 is deformable whilst the second chamber 406 is rigid or semi-rigid. The rigidity of the second chamber may, for example, protect pre-placed content 410 contained within it such as a medium (e.g., an absorbent medium) or a material for selectively binding certain molecules in the oral fluid. In some examples, the pre-placed content 410 may be a circuitry or include a circuitry in addition to a medium or material. Other features described in Figs. 1-3 may be included in mouthpiece 400, such as one way valve within inlet 403, the present or absent of outlet 404, etc.

As used herein, the term circuitry refers to (a) hardware-only circuit implementations (such as implementations with hardware components of analog and/or digital electronic circuits); and (b) a combination of circuits and software (such as firmware, drivers, applications, etc.). A circuitry may include a combination of one or more processors of any combination of types, and/or one or more memories of any combination of types. The processors, memories, and/or software, if any is present, work together to cause a circuitry to perform various functions.

A circuitry may include one or more sensors of any combination of types (e.g., accelerometers, thermometers, gyroscopes, force sensors, sensors for detecting specific chemical(s), etc.), one or more actuators, one or more transmitters, receivers, and/or transceivers, one or more antennas, one or more batteries, etc. A circuitry may store information to be retrieved and/or transmit the information wirelessly. The information may include any data collected associated with a user or a user's oral cavity(e.g., about oral fluid) and/or the analysis of such data. This definition of circuitry applies to all uses of this term herein, including in any claims.

When the mouthpiece 400 of Fig. 4a is worn by a user, biting action of the user's teeth successively compresses and releases the first chamber 402, which is deformable. This serves to pump oral fluid through the inlet 403 into the reservoir 401 and then back out through the outlet 404, passing through the rigid or semi-rigid second chamber 406 where the pre-placed content 410 is situated. This multiple chamber arrangement provides a reservoir 401 that offers both the pumping action advantage of a deformable chamber and the protective advantage of a rigid or semi-rigid chamber.

In practice, the chambers 402and 406 making up the reservoir 401 may be dimensioned and/or positioned within the mouthpiece such that the second chamber 406 does not impede the compression of the deformable first chamber 401. Fig. 4b shows a cross section of the mouthpiece 400 of Fig. 4a through the axis marked A-B where the second chamber 406 can be seen to have a much smaller depth than the deformable first chamber 402 in order that the user's teeth biting down on the mouthpiece are not significantly impeded from compressing the first chamber 402 by resistance due to the depth of the second chamber 406.

Alternatively, or additionally, the location of the second chamber 406 within the mouthpiece 400 may be chosen such that it does not impede the compression of the first chamber 402. Fig. 4c shows such a configuration as an alternative to Fig 4a, where the chamber 406a is located in a bridge portion 420 of the mouthpiece that will not be compressed by the user's teeth. In such a configuration, the second chamber 406a may have but need not have a rigid or semi-rigid construction since in use it will not be significantly compressed by the user's biting action. In some examples, both chambers 402 and 406a may have a compressible construction.

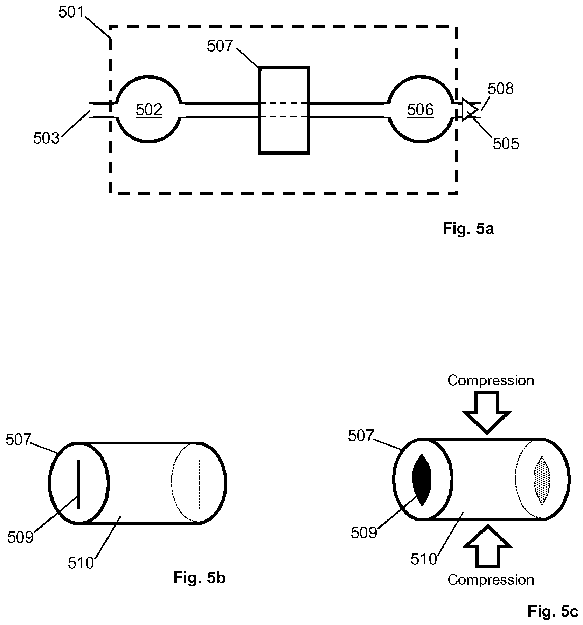

Figs. 5a-c illustrate a reservoir 501 connected to an inlet 503. The reservoir 501 comprises two chambers 502and 506, any one of which may be rigid or semi-rigid, connected in series through a compression valve 507. Prior to use, the pressure inside chamber 506 is reduced, for example by connecting a vacuum pump to a purging outlet 508 including a one way valve 505 connected to chamber 506, and pumping contents out of chamber 506 through the purging outlet 508 and valve 505 before disconnecting the pump.

Compression valve 507 may be configured such that it is closed when it is not compressed (e.g. by the user's biting action) but opens when compressed. An example of such a valve 507 is shown in Fig 5b in its uncompressed state and in Fig. 5c in its compressed state. This example of the valve 507 comprises a body 510 of elastic material such as rubber with a central slit 509 running through it along a plane parallel to an axis Y. When the valve 507 is uncompressed the two sides of the slit 509 lie against one another other, occluding valve 507. However, when the valve 507 is compressed along axis Y the two sides of the slit 509 bow out from one another creating a channel through the body 510 of the valve 507 and thus opening the valve. Upon release of the compression, the elastic body 508 of the valve 507 returns to its closed configuration, closing the channel through the slit 509 and thus closing the valve. This is just one example of a compression valve, other examples may be used in place of it.

When the user bites down on a mouthpiece containing reservoir 501, the compression valve 507 opens and the pressure difference between chambers 506 and 502 draws contents of chamber 502 into chamber 506. This in turn draws oral fluid through the inlet 503 and into chamber 502 where it is collected.

It is to be understood that the examples provided here are merely illustrative, and that in practice any suitable configuration of single or multiple reservoirs comprising single or multiple deformable, semi-rigid, and/or rigid chambers may be employed with any suitable configuration of parallel and/or series chambers.

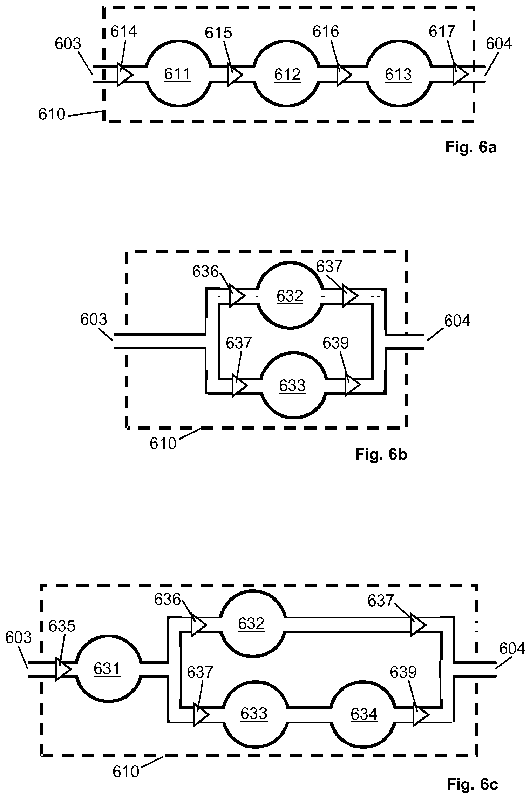

Figs. 6a-c illustrate a small number of the many different configurations that one or more reservoirs of a mouthpiece may take.

Fig. 6a illustrates a reservoir 610 connected between an inlet 603 and an outlet 604. The reservoir in this example has three chambers 611-613 connected in series with one another between the inlet 603 and outlet 604, with one way valves 614-617 between the inlet 603, each chamber 611-613, and the outlet 604 to prevent oral fluid from moving backwards through the reservoir 610. One or more of the chambers may be deformable in order to (in combination with corresponding ones the one way valves 614-617) provide a pumping action to move oral fluid forwards through the reservoir 610.

One advantage of a series arrangement of chambers such as that illustrated in Fig. 6a is that a sample of oral fluid may be passed through a series of chambers that successively treat it in different ways. For example, by passing the sample over different reagents in sequence, through a chamber containing a medium for changing the sample in some way (e.g. selective binding of certain molecules) and then into a chamber containing a preservative, or to perform successive changes on the same sample of oral fluid. If the inlet and chambers are suitably configured to draw in oral fluid at a slow rate then the series of chambers can be used to sample oral fluid collected over a long period of time, with the portions of the sample in each chamber increasing in age from the inlet to the outlet.

An example of a use case where a sequence of chambers connected in series may be beneficial is that of protein chromatographic testing. In protein chromatography different chromatography media with different protein absorbent properties are often used in sequence in order to bind different molecular targets on different surfaces. For example, to prepare changed samples for affinity chromatography followed by ion exchange chromatography. A sequence of chambers connected in series may therefore contain a medium for binding that is suitable for affinity chromatography followed by a medium for binding that is suitable for ion exchange chromatography.

Parallel collection of samples in a mouthpiece may be achieved by using multiple reservoirs each having their own inlets and (if appropriate) outlets. However, multiple chambers may be connected to shared inlets and/or (if appropriate) outlets within a single reservoir.

Fig. 6b illustrates an example in which two chambers 621 and 622 are connected in parallel within a single reservoir 620. Both chambers 621 and 622 are connected to the same inlet 603 and outlet 604 but in other examples only one of these may be shared. This example shows one way valves 623-626 separating each valve from both the inlet 603 and outlet 604 and from the other chamber 621 or 622, but any suitable arrangement of valves may be used.

Parallel arrangements of reservoirs and/or chambers within reservoirs may be useful for performing different changes (e.g. selective binding) on substantially similar samples of oral fluid, or simultaneously performing the same change more than once for greater reliability of the results. In other examples, parallel collection of samples may be useful in order to provide multiple similar samples for greater reliability of testing, for example by providing redundancy in the event that one of the chambers is contaminated or simply to obtain an overall greater volume of similar oral fluid for testing.

Fig. 6C illustrates an example of a reservoir 630 containing reservoirs 632-634 arranged in a combination of series and parallel configurations between an inlet 603 and an outlet 604. The chambers 632-634 are separated from neighbouring chambers 632-634, the inlet 603, and the outlet 604 by one-way valves 635-639. A configuration such as this may be advantageous since it permits the pumping of oral fluid through a plurality of parallel branches of rigid and/or semi-rigid chambers (e.g. that of chamber 532 and that of chambers 533 and 534) using one or more deformable chambers (chamber 531 in this example) connected in series with parallel branches.

It is to be understood that each side of a mouthpiece may comprise different numbers, types and configurations of reservoirs. It may be that one or more reservoirs is located on one side of a mouthpiece but not on the other side. In cases where the sides of the mouthpiece are not similarly arranged, dummy chambers may be included in the mouthpiece to provide a consistent feel across the sides of the mouthpiece and/or to ensure that the mouthpiece deforms appropriately when compressed by a biting action.

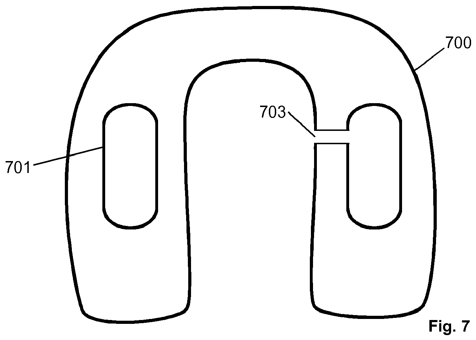

Fig. 7 illustrates an example of an asymmetric mouthpiece where a single reservoir 701 is disposed on the left side of the mouthpiece and no corresponding reservoir is disposed on the right side of the mouthpiece. For simplicity, no inlet or outlet is illustrated for reservoir 701, but in practice it will have at least an inlet. If the reservoir 701 includes one or more deformable chambers then the mouthpiece may be more readily deformable on that side, creating an uncomfortable asymmetric sensation for the user when he bites down upon it. In addition, the less-deformable right side of the mouthpiece may impede the deformation of the chamber(s) in the left side since its resilience may require the user to impart significantly greater biting pressure in order to deform both sides of the mouthpiece together. To alleviate this problem, the right side contains a dummy reservoir 702 that is constructed such that the right hand side of the mouthpiece requires a similar amount of force to deform as the left hand side. The dummy reservoir may comprise a void within the mouthpiece, which may have one or more inlet/outlet holes 703 to the exterior of the mouthpiece to allow it to be suitably compressed. In some embodiments, oral fluid may be free to enter the dummy reservoir, but would not be subsequently used for testing.

If the reservoir 701 of Fig. 7 contained one or more rigid and/or semi-rigid chambers then the dummy reservoir 702 may be constructed to include one or more rigid and/or semi-rigid structures so similarly impede deformation of the right hand side of the mouthpiece, ensuring a consistent mouth feel between the two sides.

The mouthpiece described in Figs. 1 - 7, is not limited to the examples and functions as described. The mouthpiece may be implemented or made to include different uses, including any combination of uses, whether the uses are described herein. For example, a mouthpiece may be made to use chemical sensing, electronic-based sensing, or any combination thereof, using any combination of media, materials, and/or circuitries, any of which may be placed for fitted inside and/or outside of a chamber, may be used to analyse, detect and/or collect information.

For example, instead of or in addition to a medium or material, one or more chambers described with respect to Figs. 1 - 7 may include a circuitry or different circuitries for analysing oral fluid and/or other matters in the oral cavity of a user (e.g., oral fluid in the chamber with the circuitry). One or more circuitries may be placed outside of any chamber.

A mouthpiece may be made to sense, detect, collect, or analyse one or more of, but is not limited to, the following: snoring, grinding of teeth, movements of a user, impact force (e.g., to the user's head), movements inside of a user's oral cavity, sleep stages, sleep apnea, urea in saliva, other chemicals and/or substances in saliva, etc.

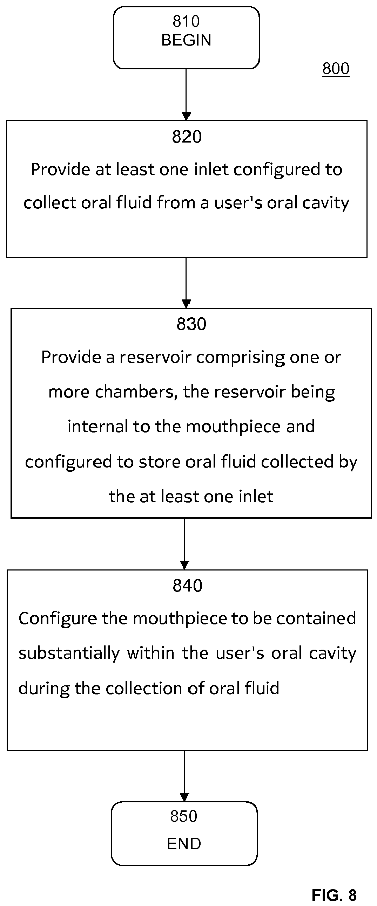

Fig. 8 illustrates a method 800 of manufacturing a mouthpiece such as those described above. The method 800 begins at step 810, after which at least one inlet is provided (820), the inlet being configured to collect oral fluid from a user's oral cavity when the mouthpiece is in use. A reservoir is then provided (820) and comprises one or more chambers, the reservoir being internal to the mouthpiece and configured to store oral fluid collected by the at least one inlet. One or more chambers may be fitted with pre-placed content, such as a medium, one or more materials, and/or a circuitry, or any combination thereof. Finally the mouthpiece is configured (840) to be contained substantially within the user's oral cavity during the collection of oral fluid. The method then ends (850).

Although the steps 820-840 are shown in Fig. 8 as occurring sequentially in a particular order, this is illustrative and in practice the steps may be performed in any appropriate order, and even simultaneously. For example, where the mouthpiece is formed at least in part through a process of moulding a material then the inlets and reservoir may be provided 820, 830 by single moulding step and thus substantially simultaneously. The step of configuring the mouthpiece 840 may be performable by manufacturing the mouthpiece according to a design that suitably dimensioned and arranged such that it is easy for a user to accommodate within the user's oral cavity during use, and may again be achieved by the moulding process.

Although moulding a uniform material such as a plastics material into an appropriate shape is one technique that might be used to manufacture the mouthpiece, other approaches may also be possible. For example, the mouthpiece may be formed from a single block of material through a milling process. Alternatively, the mouthpiece may be 3D printed using a suitable 3D printing material. Two or more suitable processes such as moulding, milling, and printing may combined in order to manufacture the mouthpiece; for example, rigid and/or semi-rigid chambers may be formed by 3D printing using a first material and the surrounding mouthpiece may then be added by moulding a second material around the printed chambers, the second material being more deformable than the first.

More generally, parts the mouthpiece may be formed around components that have been previously manufactured/assembled; for example, the reservoirs inlets and outlets (if present) may be pre-formed and a suitable material then moulded around these components in order to manufacture the mouthpiece. In this way a standard layout of internal parts may be used to manufacture mouthpieces for different users, with the final moulding being customised to each user's oral cavity- for example using a cast of the interior of the user's oral cavity.

Although various aspects of the invention are set out in the independent claims, other aspects of the invention comprise other combinations of features from the described example embodiments and/or the dependent claims with the features of the independent claims, and not solely the combinations explicitly set out in the claims.

It is also noted herein that while the above describes example embodiments of the invention, these descriptions should not be viewed in a limiting sense. Rather, there are several variations and modifications which may be made without departing from the scope of the present invention as defined in the appended claims. Furthermore, although particular combinations of features have been described in the context of specific examples, it should be understood that any of the described features may be present in any combination that falls within the scope of the claims.

## Claims

1. A mouthpiece, comprising:
at least one inlet configured to allow oral fluid from a user's oral cavity to travel into the mouthpiece; and
a first reservoir comprising one or more chambers, the first reservoir configured to store oral fluid received from the user's oral cavity through the at least one inlet,
wherein the mouthpiece is configured to be contained substantially within the user's oral cavity during the collection of oral fluid.

2. The mouthpiece of any preceding claim wherein the first reservoir comprises at least one deformable chamber.

3. The mouthpiece of claim 2 further comprising at least one outlet having a one way valve, wherein:
compression of the at least one deformable chamber forces content of the first reservoir to be purged from the mouthpiece via the at least one outlet and subsequent release of the chamber causes oral fluid to be drawn into the first reservoir via the at least one inlet.

4. The mouthpiece of any preceding claim wherein the first reservoir comprises at least one rigid or semi-rigid chamber.

5. The mouthpiece of any preceding clam, wherein the at least one inlet is dimensioned to collect the oral fluid by capillary action.

6. The mouthpiece of any preceding claim wherein the first reservoir comprises a plurality of chambers connected in series.

7. The mouthpiece of any preceding claim, wherein the first reservoir comprises a plurality of chambers connected in parallel.

8. The mouthpiece of any preceding claim, further comprising a second reservoir and a plurality of inlets, each of the first and second reservoirs being configured to store oral fluid collected by a mutually exclusive set of the plurality of inlets.

9. The mouthpiece of claim 8, wherein the first and second reservoirs each contain a different medium, at least one of which is for changing a sample of oral fluid in each respective chamber in a different way

10. The mouthpiece of any preceding claim, wherein at least one chamber contains an absorbent medium for retaining oral fluid in the chamber or a circuitry for analysing oral fluid in the chamber.

11. The mouthpiece of any preceding claim, wherein at least one chamber contains a medium for changing a sample of oral fluid in the chamber.

12. The mouthpiece of claim 11, wherein:
the first reservoir comprises a plurality of chambers; and
at least two of the plurality of chambers each contain a different medium for changing a sample of oral fluid in each respective chamber in a different way.

13. The mouthpiece of claim 10 or claim 11, wherein a medium in one of the plurality of chambers comprises a binding medium for selectively binding one or more molecules present in the sample of oral fluid or comprises a circuitry.

14. The mouthpiece of any preceding claim, further comprising at least one dummy chamber for providing a symmetrical mouth feel or a circuitry for analysing oral fluid.

15. A method of manufacturing a mouthpiece, the method comprising: providing at least one inlet configured to collect oral fluid from a user's oral cavity;
providing a reservoir comprising one or more chambers, the reservoir being internal to the mouthpiece and configured to store oral fluid collected by the at least one inlet; and
configuring the mouthpiece to be contained substantially within the user's oral cavity during the collection of oral fluid.
